# EUROPEAN PATENT APPLICATION

(11) **EP 1 743 889 A1**
(43) Date of publication of application: **17.01.2007**
(21) Application number: 05384022.9
(22) Date of filing: 15.07.2005
(51) Int. Cl.: C07D 231/06, C07D 403/12

(54) **Sustituted pyrazoline compounds, having predetermined stereochemistry, for reducing triglycerides in blood**

(71) Applicant: Laboratorios del Dr. Esteve S.A., 08041 Barcelona (ES)
(72) Inventor: Buschmann, Helmut Heinrich, 08960 Sant Just Desvern (Barcelona) (ES); Torrens Jover, Antonio, 08221 Terrasa (Barcelona) (ES); Yenes Minguez, Susana, 08750 Molins de Rei, Barcelona (ES); Mas Prio, José, 08191-Rubi, Barcelona (ES); Quintana Ruiz, Jordi Ramón, 08391-Tiana, Barcelona (ES); Dordal Zueras, Alberto, 08016 Barcelona (ES); Cuberes-Altisen, Maria Rosa, San Cugat del Valles, Barcelona (ES)

(57) **Abstract**

The present invention relates to substituted pyrazoline compounds, methods for their preparation, medicaments comprising these compounds as well as their use for the preparation of a medicament for the treatment of humans and animal.

## Description

The present invention relates to substituted pyrazoline compounds, methods for their preparation, medicaments comprising these compounds as well as their use for the preparation of a medicament for the treatment of humans and animals.

Triglycerides are the chemical form in which most fat exists in food as well as in the body. Triglycerides are present in blood plasma and, in association with cholesterol, form the plasma lipids. Triglycerides in blood plasma are derived from fats consumed directly or are synthesized from e.g. carbohydrates. Superfluous food intake is converted to triglycerides and transported to fat cells to be stored. Elevated triglycerides may also be a consequence of disease states, such as untreated diabetes mellitus. Excess of triglycerides in plasma (hypertriglyceridemia) is linked to the occurrence of coronary artery disease and possibly other disorders.

Therefore, compounds, which have an effect on triglycerides, especially in blood plasma are useful in the prevention and/or treatment of related disorders.

Thus, it was an object of the present invention to provide novel compounds for use as active substances in medicaments, which are suitable for the regulation especially the reduction of triglyceride levels in the blood plasma.

Said object was achieved by providing the substituted pyrazoline compounds of general formula I given below, their stereoisomers, corresponding salts and corresponding solvates thereof.

It has been found that these compounds have a marked effect on the level of triglycerides in the blood plasma.

Thus, in one of its aspects the present invention relates to a substituted pyrazoline compound of general formula 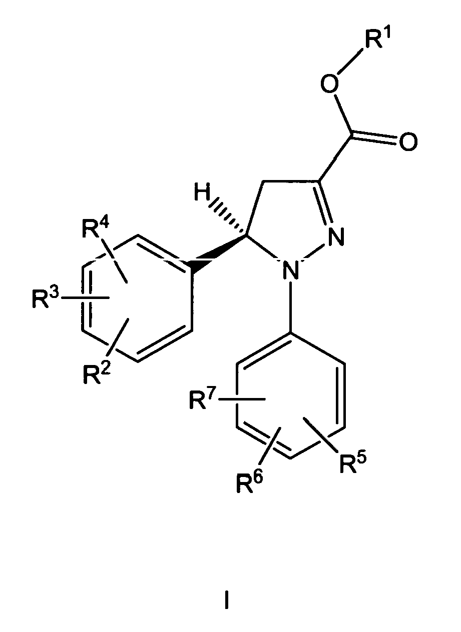 wherein
R¹ represents hydrogen or a linear or branched, substituted or unsubstituted, saturated or unsaturated C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵ and R⁶ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
R⁷ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof.

It is very preferred if to these compounds according to formula I the following proviso (disclaimer) applies:
with the proviso that
if R¹ and R⁷ are H and R⁵ and R⁶ both represent Cl in the 3- and 4-position of the phenyl ring neither of R², R³ and R⁴ may represent F in the 4-position of the phenyl ring if the other two of R², R³ and R⁴ both represent H.

These compounds had a surprising effect on the blood levels of diet relevant substances, e.g. Triglycerides.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl. C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆₋alkoxy, a C₃₋₈-cycloalkoxy, a C₃₋₈-cycloalkyl or a C₂₋₆-alkylene.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions. Especially this covers any "physiologically acceptable salt".

The term "physiologically acceptable salt" to be understood as being equivalent and interchangeable with "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of:
hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates (mono- and multihydrates) and alcoholates, e.g. methanolate and ethanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

In a preferred embodiment of the invention for a compound according to formula I at least one of R², R³ or R⁴ represents hydrogen, while at least one of R², R³ or R⁴ is different from hydrogen.

In a preferred embodiment of the invention for a compound according to formula I R⁷ represents hydrogen.

In a preferred embodiment of the invention for a compound according to formula I R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

In a preferred embodiment of the invention for a compound according to formula I R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

In a preferred embodiment of the invention for a compound according to formula I R² represents a chlorine atom in the 4-position of the phenyl ring, while R³ and R⁴ represent hydrogen.

In a preferred embodiment of the invention for a compound according to formula I R⁵ and R⁶ each represent a chlorine atoms in the 2- and 4-position of the phenyl ring, while R⁷ represents hydrogen.

In a preferred embodiment of the invention for a compound according to formula I R¹ represents hydrogen, methyl or ethyl, preferably hydrogen.

In a highly preferred further aspect of the invention the compound of general formula I is represented by a compound of general formula II 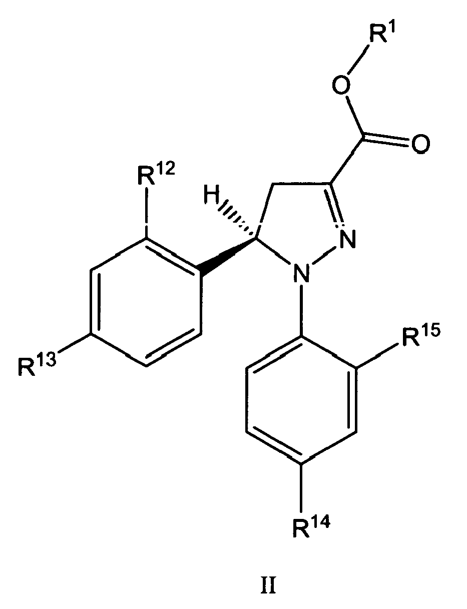 wherein
R¹ represents hydrogen or a linear or branched, substituted or unsubstituted, saturated or unsaturated, C₁₋₄-alkyl group,
R¹² or R¹³ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, methyl, ethyl, F, Cl, Br and CF₃, optionally in form of a corresponding N-oxide thereof, or a corresponding salt thereof, or a corresponding solvate thereof.

In a preferred embodiment of the invention for a compound according to formula II R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

In a preferred embodiment of the invention for a compound according to formula II R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

In a preferred embodiment of the invention for a compound according to formula II R¹³ represents Cl and R¹² represents hydrogen.

In a preferred embodiment of the invention for a compound according to formula II R¹⁴ and R¹⁵ each represent Cl.

In a preferred embodiment of the invention for a compound according to formula II R¹ represents hydrogen, methyl or ethyl, preferably hydrogen.

In another preferred embodiment the compound according to formula I or II is:
(R)-5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.

Another preferred embodiment of the invention covers also any prodrug of the compounds of the invention described above as well as any medicament comprising this and any use thereof; especially including their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In another preferred embodiment of the invention it also covers mixtures of the compounds of the invention (according to formulas I and II) described above with their corresponding opposite enantiomer according to formula XX 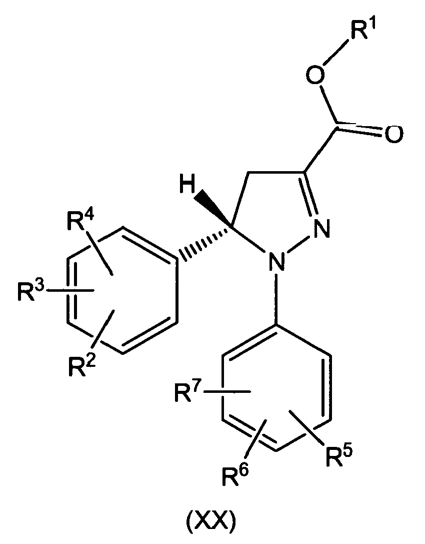 in any suitable ratio (excluding a racemic mixture), as well as any combination and medicament comprising this and any use thereof described below.

Another aspect of the invention is a combination of compounds comprising at least one substituted pyrazoline compound of general formula I 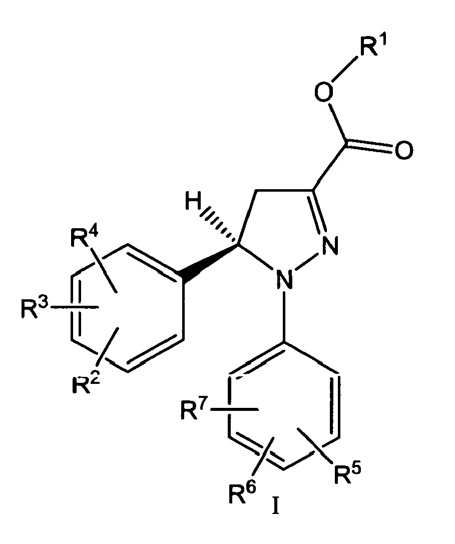 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof; and at least one substituted pyrazoline compound of general formula X 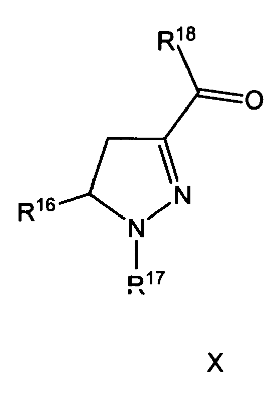 wherein
R¹⁶ represents an optionally at least mono-substituted phenyl group,
R¹⁷ represents an optionally at least mono-substituted phenyl group,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an - NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R²¹-moiety, or an-NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the combination of compounds according to the invention for a compound according to formula I at least one of R², R³ or R⁴ represents hydrogen, while at least one of R², R³ or R⁴ is different from hydrogen.

In a preferred embodiment of the combination of compounds according to the invention for a compound according to formula I at least on of R⁵, R⁶ or R⁷ represents hydrogen, while at least one R⁵, R⁶ or R⁷ is different from hydrogen.

In a preferred embodiment of the combination of compounds according to the invention for a compound according to formula I R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

In a preferred embodiment of the combination of compounds according to the invention for a compound according to formula I R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃.

In a preferred embodiment of the combination of compounds according to the invention for a compound according to formula I R² represents a chlorine atom in the 4-position of the phenyl ring, while R³ and R⁴ represent hydrogen.

In a preferred embodiment of the combination of compounds according to the invention for a compound according to formula I R⁵ and R⁶ each represent a chlorine atom in the 2- and 4-position of the phenyl ring, while R⁷ represents hydrogen.

In a preferred embodiment of the combination of compounds according to the invention for a compound according to formula I R¹ represents hydrogen, methyl or ethyl, preferably hydrogen.

In a preferred embodiment of the combination of compounds according to the invention the compound of general formula I is represented by a compound of general formula II 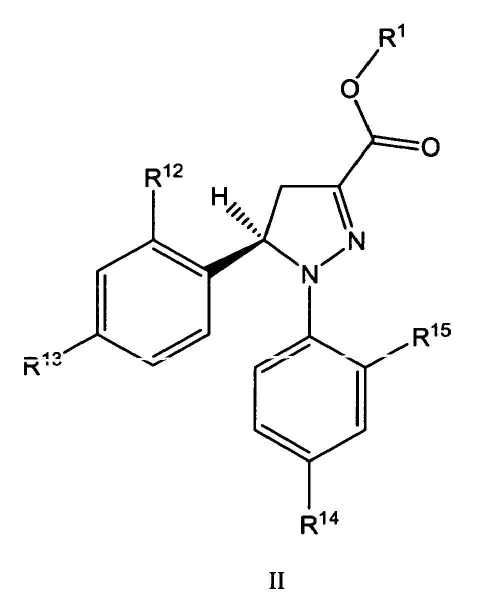 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof.

In a preferred embodiment of the combination of compounds according to the invention for a compound according to formula II R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

In a preferred embodiment of the combination of compounds according to the invention for a compound according to formula II R¹⁴, and R¹⁵ independently of each other represent hydrogen, a linear or branched C₁₋₆-alkyl group, a halogen atom, or CF₃, preferably R¹⁴ and R¹⁵ independently of each other represent hydrogen, methyl, ethyl, F, Cl, Br and CF₃.

In a preferred embodiment of the combination of compounds according to the invention for a compound according to formula II R¹³ represents Cl and R¹² represents hydrogen.

In a preferred embodiment of the combination of compounds according to the invention for a compound according to formula II R¹⁴ and R¹⁵ each represent Cl.

In a preferred embodiment of the combination of compounds according to the invention for a compound according to formula II R¹ represents hydrogen, methyl or ethyl, preferably hydrogen.

In a preferred embodiment of the combination of compounds according to the invention the compound according to formula I or II is:
(R)-5-(4-chloro-phenyl)-1-(2,4-dichlorophenyl)-4,5-dihydro-1 H-pyrazol-3-carboxylic acid,
optionally in the form of a corresponding N-oxide, a corresponding salt or a corresponding solvate.

In a preferred embodiment of the combination of compounds according to the invention for a compound according to formula X R¹⁶ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂,-(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R¹⁶ represents a phenyl group, which is optionally substituted by one or more substituents selected from the group consisting of methyl, ethyl, F, Cl, Br and CF₃, more preferably R¹⁶ represents a phenyl group, which is mono-substituted with a chlorine atom in the 4-position.

In a preferred embodiment of the combination of compounds according to the invention for a compound according to formula X R¹⁷ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH. NO₂,-(C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R¹⁷ represents a phenyl group, which is optionally substituted by one or more substituents independently selected from the group consisting of methyl, ethyl, F, Cl, Br and CF₃, more preferably R¹⁷ represents a phenyl group, which is di-substituted with two chlorine atoms in its 2- and 4-position.

In a preferred embodiment of the combination of compounds according to the invention for a compound according to formula X R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an ―NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an -NR¹⁹R ²⁰-moiety, more preferably R¹⁸ represents a pyrrolidinyl group, a piperidinyl group or a piperazinyl group, whereby each of these groups may be substituted with one or more C₁₋₆-alkyl groups, or an ―NR¹⁸R¹⁹-moiety.

In a preferred embodiment of the combination of compounds according to the invention for a compound according to formula X R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an-SO₂-R²¹-moiety, or an ―NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an -SO₂-R²¹-moiety, or an -NR²²R²³-moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an -SO₂-R²¹-moiety, or an -NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

In a preferred embodiment of the combination of compounds according to the invention for a compound according to formula X R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R²¹ represents a C₁₋₆-alkyl group, a saturated, optionally at least mono-substituted cycloaliphatic group, which may be condensed with a mono- or polycyclic ring-system, or a phenyl group, which is optionally substituted with one or more C₁₋₆ alkyl groups.

In a preferred embodiment of the combination of compounds according to the invention for a compound according to formula X R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

In a preferred embodiment of the combination of compounds according to the invention the compound according to general formula X is represented by a structure wherein
R¹⁶ represents a phenyl ring, which is mono-substituted with a halogen atom, preferably a chlorine atom, in its 4-position,
R¹⁷ represents a phenyl ring, which is di-substituted with two halogen atoms, preferably chlorine atoms, in its 2- and 4-position,
R¹⁸ represents a pyrrolidinyl group, a piperidinyl group, a piperazinyl group, a homo-piperazinyl group, a morpholinyl group, or an ―NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the combination of compounds according to the invention the combination of compounds comprises at least one compound according to formula X selected from the group consisting of:
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
   optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the combination of compounds according to the invention the combination of compounds comprises at least one compound according to formula X selected from
(R)-5-(4-chloro-phenyl)-1-(2,4-dichlorophenyl)-4,5-dihydro-1H-pyrazol-3-carboxylic acid,
and
- (R)-N-piperidinyl-5-(4-chloro-phenyl)-1-(2,4-dichlorophenyl)-4,5-dihydro-1 H-pyrazol-3-carboxamide; or
- (S)-N-piperidinyl-5-(4-chloro-phenyl)-1-(2,4-dichlorophenyl)-4,5-dihydro-1 H-pyrazol-3-carboxamide; or
- mixtures of these, especially
- (rac)-N-piperidinyl-5-(4-chloro-phenyl)-1-(2,4-dichlorophenyl)-4,5-dihydro-1 H-pyrazol-3-carboxamide;
   each optionally in the form of a corresponding N-oxide, a corresponding salt or a corresponding solvate.

In another aspect the present invention also provides a process for the preparation of substituted pyrazoline compounds of general formula I or II, wherein R¹ is hydrogen, given above, in that at least one benzaldehyde compound of general formula III 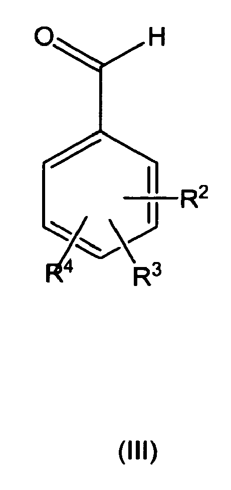 wherein R², R³ and R⁴ have the meaning mentioned above, is reacted with a pyruvate compound of general formula (IV) 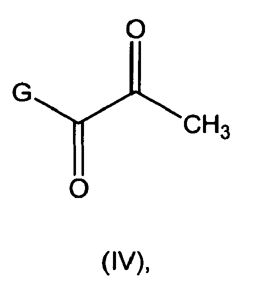 wherein G represents an OR group with R being a branched or unbranched C₁₋₆ alkyl radical or G represents an O⁻K group with K being a cation, preferably an anorganic kation, more preferably an alkali metal kation, most preferably sodium, to yield a compound of general formula (V) 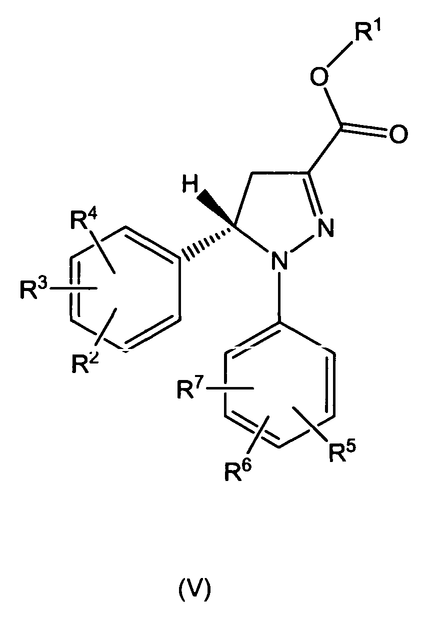 which is optionally isolated and/or optionally purified, and which is reacted with an optionally substituted phenyl hydrazine of general formula (VI) 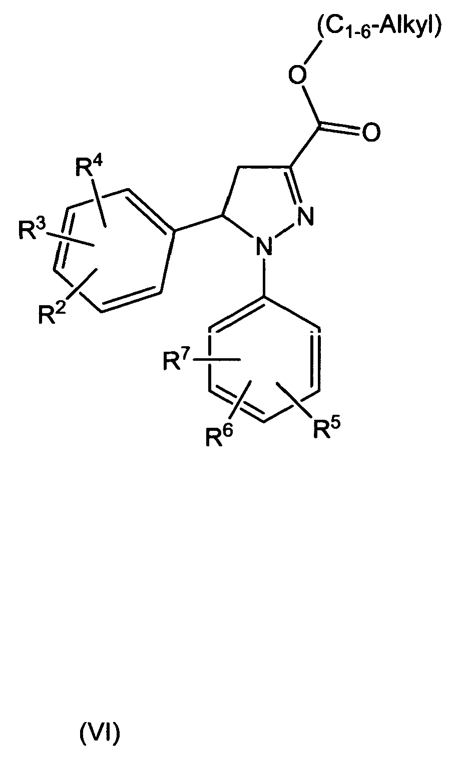 or a corresponding salt thereof, wherein R⁵, R⁶ and R⁷ have the meaning mentioned above, under inert atmosphere, to yield a compound of general formula (VII) 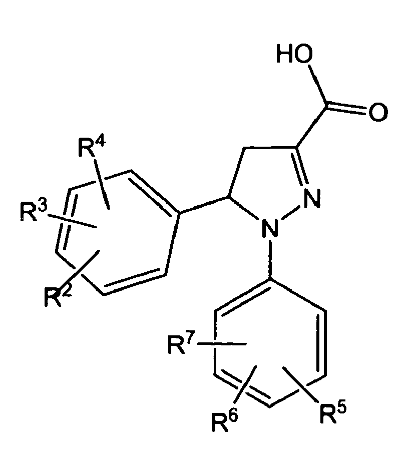 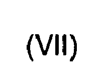 wherein R², R³, R⁴, R⁵, R⁶ and R⁷ have the meaning as given above, which is optionally isolated and/or optionally purified, and optionally esterified to an alkyl-ester if in the substituted pyrazoline compound of general formula I or II according to the invention R¹ is a linear or branched, substituted or unsubstituted, saturated or unsaturated, C₁₋₄-alkyl group.

This is followed by addition of chiral base.

The inventive process is also illustrated in scheme I given below:

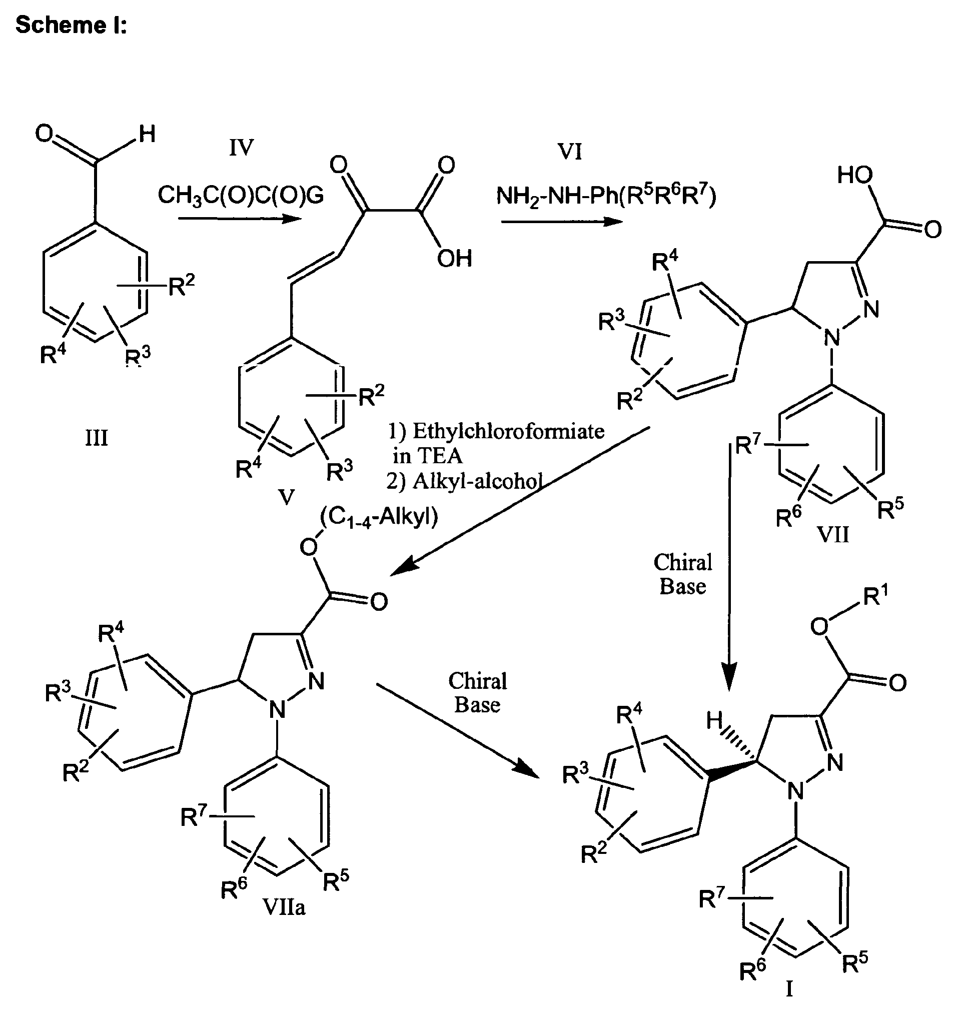

The reaction of the benzaldehyde compound of general formula III with a pyruvate compound of general formula IV is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably said reaction is carried out in a protic reaction medium such as a C₁₋₄ alkyl alcohol or mixtures of these.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula III with a pyruvate compound of general formula IV is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (V) with an optionally substituted phenyl hydrazin of general formula (VI) is preferably carried out in a suitable reaction medium such as C₁₋₄-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VII) may be activated for further reactions by the introduction of a suitable leaving group according to conventional methods well known to those skilled in the art. Preferably the compounds of general formula (VII) are transferred into an acid chloride, an acid anhydride, a mixed anhydride, a C₁₋₄ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VII) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VII) is a mixed anhydride, said anhydride may preferably be prepared, for example, by reaction of the corresponding acid of general formula (VII) with ethyl chloroformiate in the presence of a base such as triethylamine or pyridine, in a suitable solvent.

Following that the activated compound can be reacted with an alkyl-alcohol to arrive at compounds according to general formulas VIIa being the equivalent of compounds of general formula I with R¹ being a linear or branched, substituted or unsubstituted, C₁₋₄-alkyl group.

Finally compounds VII or VIIa are treated with a suitable chiral base, preferably (+)-Cinchonine or R-(+)-1-Phenylethylamine, to arrive at compounds according to general formula I.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula I or II themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis.

In a further aspect the present invention also provides a process for the preparation of salts of substituted pyrazoline compounds of general formula I or II and stereoisomers thereof, wherein at least one compound of general formula I or II having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula I or II or stereoisomers thereof, wherein at least one compound of general formula I or II having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted pyrazoline compounds of general formula I or II, of corresponding stereoisomers, of corresponding N-oxides or of corresponding salts thereof may also be obtained by standard procedures known to those skilled in the art.

Substituted pyrazoline compounds of general formula I or II, which comprise nitrogen-atom containing saturated, unsaturated or aromatic rings may also be obtained in the form of their N-oxides by methods well known to those skilled in the art.

The purification and isolation of the inventive substituted pyrazoline compounds of general formula I or II, of a corresponding stereoisomer, or salt, or solvate or any intermediate thereof may, if required, be carried out by conventional methods known to those skilled in the art, e.g. chromatographic methods or recrystallization.

As a reference on stereochemistry and the standard methods for seperation reference is made to Eliel and Wilen, Stereochemistry of organic Compounds, John Wiley & Sons Inc., 1994, included by reference. Preferred methods include MPLC, HPLC, simulated moving batch and imprinted polymers.

The substituted pyrazoline compounds of general formula I and II given below, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula (I) and (II) given below, stereoisomers thereof, N-oxides thereof, corresponding salts and corresponding solvates have the property to regulate triglyceride levels in blood plasma.

The combination of compounds comprising substituted pyrazoline compounds of general formula (I) and (II) given below, their stereoisomers, corresponding N-oxides, corresponding salts thereof and corresponding solvates and of substituted pyrazoline compounds of general formula (X) given below, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula X given below, stereoisomers thereof, N-oxides thereof, corresponding salts and corresponding solvates have a high affinity to cannabinoid receptors, particularly cannabinoid 1 (CB₁)-receptors, i.e. they act as antagonists on these receptors. In particular these pyrazoline compounds show litte or no development of tolerance during treatment particularly with respect to food intake. After ending the treatment with the pyrazoline compounds, reduced increase of body weight is found compared to the pre-treatment level.

Thus, another aspect of the present invention relates to a Medicament comprising at least one substituted pyrazoline compound of general formula I or II according to the invention and optionally one or more pharmaceutically acceptable excipients.

Thus, another aspect of the present invention relates to a Medicament comprising at least one substituted pyrazoline compound of general formula I 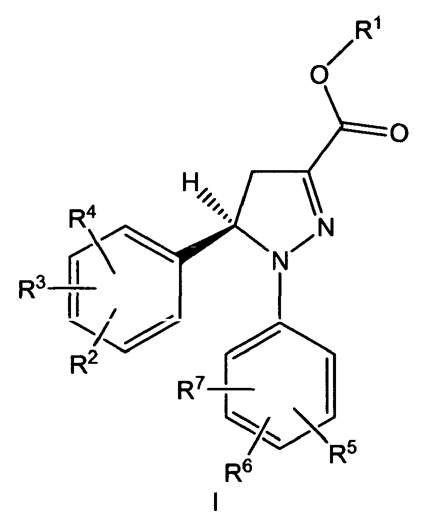 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof;
and optionally one or more pharmaceutically acceptable excipients.

In an embodiment of the medicament according to the invention for the compound according to formula I at least one of R², R³ or R⁴ represents hydrogen, while at least one of R², R³ or R⁴ is different from hydrogen.

In an embodiment of the medicament according to the invention for the compound according to formula I at least one of R⁵, R⁶ or R⁷ represents hydrogen, while at least one R⁵, R⁶ or R⁷ is different from hydrogen.

In an embodiment of the medicament according to the invention for the compound according to formula I R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

In an embodiment of the medicament according to the invention for the compound according to formula I R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃.

In an embodiment of the medicament according to the invention for the compound according to formula I R² represents a chlorine atom in the 4-position of the phenyl ring, while R³ and R⁴ represent hydrogen.

In an embodiment of the medicament according to the invention for the compound according to formula I R⁵ and R⁶ each represent a chlorine atoms in the 2- and 4-position of the phenyl ring, while R⁷ represents hydrogen.

In an embodiment of the medicament according to the invention for the compound according to formula I R¹ represents hydrogen, methyl or ethyl, preferably hydrogen.

In an embodiment of the medicament according to the invention the compound according to formula I is represented by a compound of general formula (II) 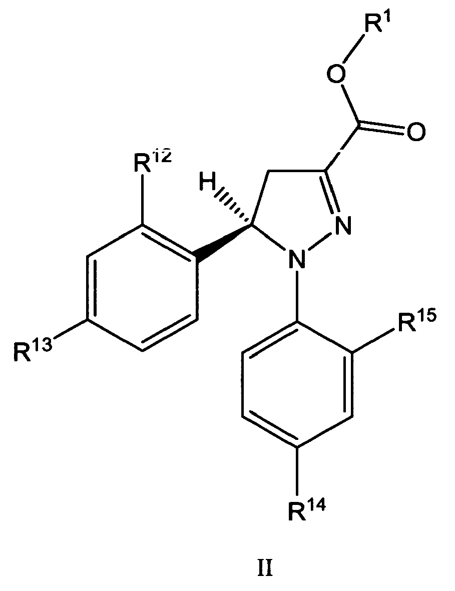 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof.

In an embodiment of the medicament according to the invention for the compound according to formula II R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

In an embodiment of the medicament according to the invention for the compound according to formula II R¹⁴, and R¹⁵ independently of each other represent hydrogen, a linear or branched C₁₋₆-alkyl group, a halogen atom, or CF₃, preferably R¹⁴ and R¹⁵ independently of each other represent hydrogen, methyl, ethyl, F, Cl, Br and CF₃.

In an embodiment of the medicament according to the invention for the compound according to formula II R¹³ represents Cl and R¹² represents hydrogen.

In an embodiment of the medicament according to the invention for the compound according to formula II R¹⁴ and R¹⁵ each represent Cl.

In an embodiment of the medicament according to the invention for the compound according to formula II R¹ represents hydrogen, methyl or ethyl, preferably hydrogen.

In an embodiment of the medicament according to the invention the compound according to formulas I or II is selected from the group consisting of:
(R)-5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.

Another aspect of the invention is a medicament comprising at least one combination of compounds according to the invention and optionally one or more pharmaceutically acceptable excipients.

In an embodiment of the medicaments according to the invention the medicament is for the regulation of triglyceride levels in the blood plasma and for the prophylaxis and/or treatment of disorders of the central nervous system, especially stroke, of disorders of the cardiovascular system and of of food intake disorders, preferably bulimia, anorexia, cachexia, obesity, type II diabetus mellitus (non-insuline dependent diabetes mellitus), preferably obesity and diabetis.

In an embodiment of the medicament comprising the compound according to formula I or II according to the invention the medicament is for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the medicament comprising the combination according to the invention the medicament is for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the medicaments according to the invention the medicament is for the prophylaxis and/or treatment of food intake disorders, preferably bulimia, anorexia, cachexia, obesity, type II diabetus mellitus (non-insuline dependent diabetes mellitus), preferably obesity.

In an embodiment of the medicaments according to the invention the medicament is for the prophylaxis and/or treatment of psychosis.

In an embodiment of the medicaments according to the invention the medicament is for the prophylaxis and/or treatment of alcohol abuse and/or addiction, nicotine abuse and/or addiction, drug abuse and/or addiction and/or medicament abuse and/or addiction, preferably drug abuse and/or addiction and/or nicotine abuse and/or addiction.

In an embodiment of the medicaments according to the invention the medicament is for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Said medicaments may also comprise any combination of one or more of the substituted pyrazoline compounds of general formula I given above, stereoisomers thereof, corresponding N-oxides thereof, physiologically acceptable salts thereof or physiologically acceptable solvates thereof.

Particularly preferably said medicaments are suitable for the prophylaxis and/or treatment of alcohol abuse, drug abuse and/or medicament abuse, preferably drug abuse and the treatment of obesity.

Medicaments and/or drugs, which are frequently the subject of misuse include opioids, barbiturates, cannabis, cocaine, amphetamines, phencyclidine, hallucinogens and benzodiazepines.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils.

Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Another preferred aspect of the invention is the use of at least one substituted pyrazoline compound of formula I or II according to the invention or at least one combination of compounds according to the invention (and optionally one or more pharmaceutically acceptable excipients), for the preparation of a medicament for the regulation of triglyceride levels in the blood plasma and for the prophylaxis and/or treatment of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formaula I or II according to the invention (and optionally one or more pharmaceutically acceptable excipients,) for the preparation of a medicament for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is the use of at least one combination of compounds according to the invention (and optionally one or more pharmaceutically acceptable excipients,) for the preparation of a medicament for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is the use of at least one substituted pyrazoline compound of formula I or II according to the invention or at least one combination of compounds according to the invention (and optionally one or more pharmaceutically acceptable excipients), for the preparation of a medicament for the prophylaxis and/or treatment of food intake disorders, preferably bulimia, anorexia, cachexia, obesity, type II diabetus mellitus (non-insuline dependent diabetes mellitus), preferably obesity.

Another preferred aspect of the invention is the use of at least one substituted pyrazoline compound of formula I or II according to the invention or at least one combination of compounds according to the invention (and optionally one or more pharmaceutically acceptable excipients), for the preparation of a medicament for the prophylaxis and/or treatment of psychosis.

Another preferred aspect of the invention is the use of at least one substituted pyrazoline compound of formula I or II according to the invention or at least one combination of compounds according to the invention (and optionally one or more pharmaceutically acceptable excipients), for the preparation of a medicament for the prophylaxis and/or treatment of alcohol abuse and/or addiction, nicotine abuse and/or addiction, medicament abuse and/or addiction and/or drug abuse and/or addiction, preferably drug abuse and/or addiction or nicotine abuse and/or addiction.

Another preferred aspect of the invention is the use of at least one substituted pyrazoline compound of formula I or II according to the invention or at least one combination of compounds according to the invention (and optionally one or more pharmaceutically acceptable excipients), for the preparation of a medicament for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Another preferred aspect of the invention is a method of regulating triglyceride levels in the blood plasma, a method for treating disorders of the central nervous system, especially stroke, disorders of the cardiovascular system or of food intake disorders, especially bulimia, anorexia, cachexia, obesity, type II diabetus mellitus (non-insuline dependent diabetes mellitus), preferably obesity and diabetis, comprising administering to a subject, preferably a human, a therapeutically effective amount of one or more of the pyrazoline compounds or mixtures as described herein.

Another preferred aspect of the invention is a method of treating disorders of the central nervous system, disorders of the immune system, disorders of the cardiovascular system, disorders of the endocrinous system, disorders of the respiratory system, disorders of the gastrointestinal tract or reproductive disorders, comprising administering to a subject, preferably a human, a therapeutically effective amount of one or more of the pyrazoline compounds or mixtures as described herein.

Another preferred aspect of the invention is a method of treating psychosis, comprising administering to a subject, preferably a human, a therapeutically effective amount of one or more of the pyrazoline compounds or mixtures as described herein.

Another preferred aspect of the invention is a method for treating alcohol abuse and/or addiction, nicotine abuse and/or addiction, medicament abuse and/or addiction and/or drug abuse and/or addiction, preferably drug abuse and/or addiction or nicotine abuse and/or addiction, comprising administering to a subject, preferably a human, a therapeutically effective amount of one or more of the pyrazoline compounds or mixtures as described herein.

Another preferred aspect of the invention is a method of treating one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit, comprising administering to a subject, preferably a human, a therapeutically effective amount of one or more of the pyrazoline compounds or mixtures as described herein.

Another preferred aspect of the invention is the use of at least one substituted pyrazoline compound of general formula I 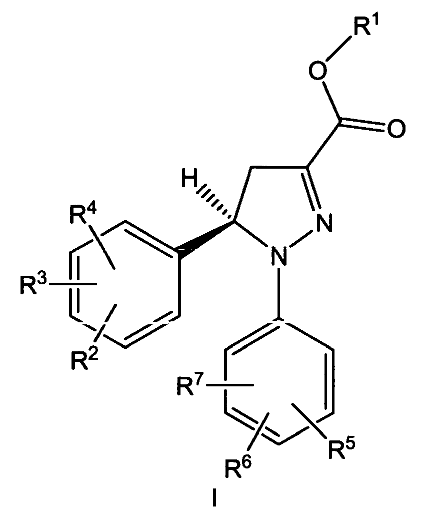 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof;
and optionally one or more pharmaceutically acceptable excipients, for the preparation of a medicament for the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use for the manufacture of a medicament of at least one substituted pyrazoline compound of formula I for which at least one of R², R³ or R⁴ represents hydrogen, while at least one of R², R³ or R⁴ is different from hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which at least on of R⁵, R⁶ or R⁷ represents hydrogen, while at least one R⁵, R⁶ or R⁷ is different from hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃ for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃ for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which R² represents a chlorine atom in the 4-position of the phenyl ring, while R³ and R⁴ represent hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which R⁵ and R⁶ each represent a chlorine atoms in the 2- and 4-position of the phenyl ring, while R⁷ represents hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula I for which R¹ represents hydrogen, methyl or ethyl, preferably hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formula wherein the compound of general formula (I) is represented by a compound of general formula (II) 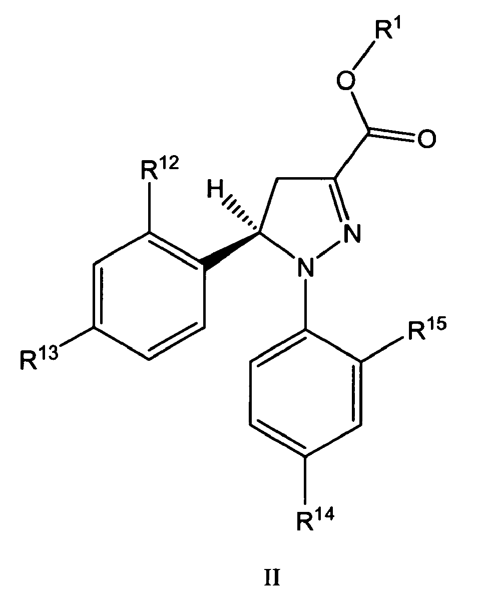 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof
for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another embodiment of the invention is the use of at least one substituted pyrazoline compound of formula II for which R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃ for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another embodiment of the invention is the use of at least one substituted pyrazoline compound of formula II for which R¹⁴, and R¹⁵ independently of each other represent hydrogen, a linear or branched C₁₋₆-alkyl group, a halogen atom, or CF₃, preferably R¹⁴ and R¹⁵ independently of each other represent hydrogen, methyl, ethyl, F, Cl, Br and CF₃ for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another embodiment of the invention is the use of at least one substituted pyrazoline compound of formula II for which R¹³ represents Cl and R¹² represents hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred embodiment of the invention is the use of at least one substituted pyrazoline compound of formula II for which R¹⁴ and R¹⁵ each represent Cl for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred embodiment of the invention is the use of at least one substituted pyrazoline compound of formula II for which R¹ represents hydrogen, methyl or ethyl, preferably hydrogen for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another embodiment of the invention is the use of at least one substituted pyrazoline compound of formula I or II for which the compound according to formulas I or II is selected from the group consisting of:
(R)-5-(4-chloro-phenyl)-1-(2,4-dichlorophenyl)-4,5-dihydro-1 H-pyrazol-3-carboxylic acid,
optionally in the form of a corresponding N-oxide, a corresponding salt or a corresponding solvate;
   for the manufacture of a medicamentfor the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Also particularly preferred is the use of at least one of the pyrazoline compounds as defined herein and optionally one or more pharmaceutically acceptable excipients, for the preparation of a medicament for the treatment of metabolic syndrome.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.
The metabolic syndrome is characterized by an interaction of several physiological parameters such as triglycerides, lipids, blood pressure, glucose levels and insuline levels.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Another aspect of the invention is the use of one or more pyrazoline compounds as defined herein for the manufacture of a medicament for improvent of cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia.

Another aspect of the invention is the use of one or more pyrazoline compounds as defined herein for the manufacture of a medicament for the treatment of the weight independent aspects of metabolic syndrome.

Another aspect of the invention is a method for improving cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia,

in a subject, preferably a human.

Another aspect of the invention is a method for treating of the weight independent aspects of metabolic syndrome.

The present invention is illustrated below with the aid of examples. These illustrations are given solely by way of example and are not intended to limit the present invention.

### Examples:

Example 0 represent a compound according to formula I or II.

### Example 0:

**(R)-5-(4-chlorophenyl)-1-(2,4-dichlorophenyl)-4,5-dihydro-pyrazole-3-carboxylic acid**

### a) 4-(4-chlorophenyl)-2-oxo-3-butenoic acid

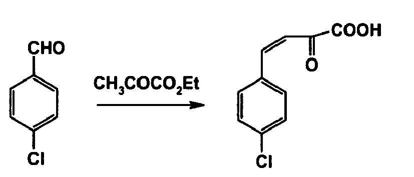

In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, cm⁻¹) : 3500-2500, 1719,3, 1686,5, 1603,4, 1587,8, 1081,9. ¹H NMR(CDCl₃, δ) : 7,4 (d, J=8,4Hz, 2H), 7,5 (d, J=16,1Hz, 1 H), 7,6 (d, J=8,4Hz, 2H), 8,1(d, J=16,1 Hz, 1 H).

### a2) 5-(4-chlorophenyl)-1-(2,4-dichlorophenyl)-4,5-dihydro-pyrazole-3-carboxylic acid

In an alternative route instead of using ethylpyruvate the salt CH₃-C(O)-C(O)-O⁻ Na⁺ (sodiumpyruvate) was used, dissolved ethanolic water.

### b) 5-(4-chlorophenyl)-1-(2,4-dichlorophenyl)-4,5-dihydro-pyrazole-3-carboxylic acid

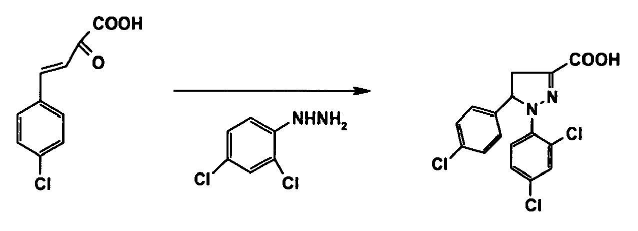

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, cm⁻¹) : 3200-2200, 1668,4, 1458, 1251,4, 1104,8. ¹H NMR (CDCl₃, δ) : 3,3 (dd, 1 H), 3,7 (dd, 1 H), 5,9 (dd, 1 H), 7,09-7,25 (m, 7H).

### c) Resolution of the enantiomers of 5-(4-chlorophenyl)-1-(2,4-dichlorophenyl)-4,5-dihydro-pyrazole-3-carboxylic acid

The resolution of the enantiomers of 5-(4-chlorophenyl)-1-(2,4-dichlorophenyl)-4,5-dihydro-pyrazole-3-carboxylic acid was carried out via reaction with the following chiral bases:
- Brucine
- Quinine
- (-)-Cinchonidine
- (+)-Cinchonine
- R-(+)-1-Phenylethylamine
- (1 R,2S)-(-)-Ephedrine hydrochloride
- (1S,2R)-(+)-Ephedrine hydrochloride.
   In each case the reactions were carried out with 0.5 and 1 equivalents of base in respect to 1 equivalent of the acid compound and by using the following solvents
- Ethanol
- Acetone
- Acetonitril
- Dioxane
- Ethylacetate
- Chloroform.

The results are summarized in the following tables. It may be understood that the aforementioned crystallisation experiments that are not reflected in the following tables did not yield crystals of the respective salts under the given conditions.

However, suitable conditions for crystallization of these salts can be determined by those skilled in the art via routine experiments.

In the following tables

Acid represents racemic 5-(4-chlorophenyl)-1-(2,4-dichlorophenyl)-4,5-dihydro-pyrazole-3-carboxylic acid

R-Acid represents the respective derivative of (R)-5-(4-chlorophenyl)-1-(2,4-dichlorophenyl)-4,5-dihydro-pyrazole-3-carboxylic acid

S-Acid represents the respective derivative of (S)-5-(4-chlorophenyl)-1-(2,4-dichlorophenyl)-4,5-dihydro-pyrazole-3-carboxylic acid

### Processes for crystallisation:

Process A: A solution of the chiral base was added on top of a solution of the racemic acid at room temperature.

Process C: A solution of the racemic acid was added on top of a solution of the chiral base. The mixture was heated to reflux and solvent was added until dissolution was complete. The solution was left to crystallisation at r.t.

Process D: The chiral base was directly added on top of a solution of the racemic acid at room temperature.

Process E: The chiral base was directly added on top of a solution of the racemic acid at reflux temperature.

Process F: The solution of the salt was evaporated to dryness. The residue was dissolved in a minimum amount of the solvent under reflux heating. The solution was left to crystallisation at r.t.

### Resolution with Brucine

| Brucine | | | | | | | | | |
|---|---|---|---|---|---|---|---|---|---|
| 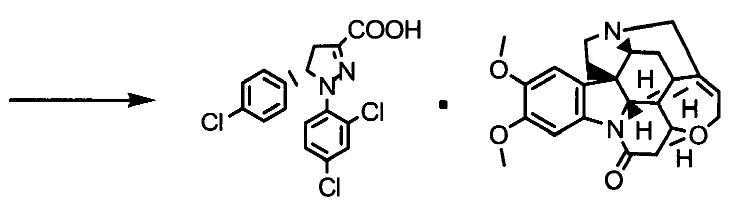 | | | | | | | | | |

| Acid g (mmol) | Eq. amine | Proc. | Solvent for crystallisation | T Cryst. | Yield 1^{st} Cryst. % | % S-Acid | % R-Acid | Yield 2^{nd} Cryst. % | % S-Acid |
|---|---|---|---|---|---|---|---|---|---|
| 0,4 g (1,09 mmol) | 0,5 | A | 8 ml acetone | r.t. | 44,6 | 98,8 | 1,2 | 5,6% | 99,4 |
| 0,4 g (1,09 mmol) | 0,5 | A | 11,5 ml acetonitrile | r.t. | 50,7 | 47,5 | 52,4 | | |
| 0,4 g (1,09 mmol) | 1 | A | 17 ml acetonitrile | r.t. | 25,6 | 45,7 | 54,3 | | |

### Resolution with Quinine

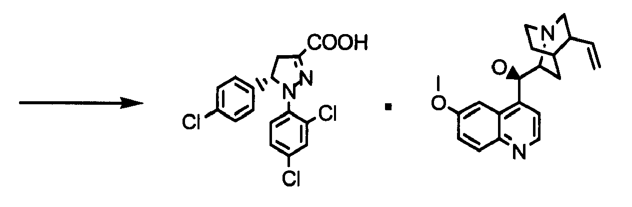

| Acid g (mmol) | Eq. amine | Proc. | Solvent for crystallisation | T Crys. | Yield 1^{st} Cryst. % | % S-Acid | % R-Acid |
|---|---|---|---|---|---|---|---|
| 0,4 g (1,09 mmol) | 1 | A | 8 ml dioxane | r.t. | 49,46 | 91,6 | 8,3 |
| 0,4 g (1,09 mmol) | 1 | F | 15ml acetonitrile | r.t. | 26 | 94,4 | 5,5 |
| 0,4 g (1,09 mmol) | 1 | F | 2ml ethylacetate | r.t. | 25 | 96,7 | 3,3 |
| 0,4 g (1,09 mmol) | 0,5 | A | 4ml dioxane | r.t. | 38,5 | 97,5 | 2,5 |

### Resolution with (-)-Cinchonidine

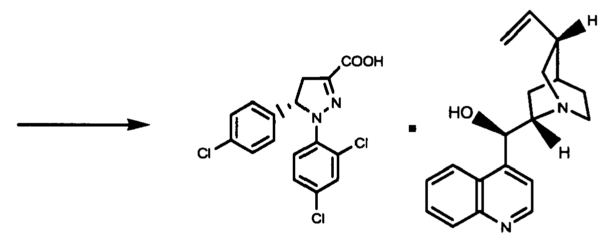

| Acid g (mmol) | Eq. amine | Proc. | Solvent for crystallisation | T Cryst. | Yield 1^{st} Cryst. % | % S-Acid | % R-Acid |
|---|---|---|---|---|---|---|---|
| 0,4 g (1,09 mmol) | 1 | F | 2ml dioxane | r.t. | 31 | 94,4 | 5,6 |
| 0,4 g (1,09 mmol) | 1 | F | 19ml Ethylacetate | r.t. | 28,5 | 95,8 | 4,2 |
| 0,4 g (1,09 mmol) | 1 | F | 20ml acetone | r.t. | 19,6 | 96,9 | 3,1 |
| 0,4 g (1,09 mmol) | 1 | F | 24ml acetonitrile | r.t. | 42 | 85,8 | 14,1 |

### Resolution with (+)-Cinchonine

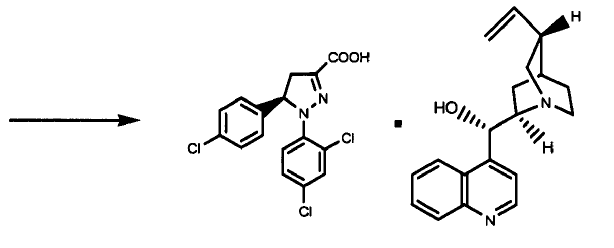

| Acid | Eq. amine | Proc. | Solvent for crystallisation | T Cryst . | Yield 1^{st} Cryst. % | % S-Acid | % R-Acid |
|---|---|---|---|---|---|---|---|
| 0,4 (1,09 mmol) | 1 | C | 50 ml acetone | r.t. | 32,8 | 7,42 | 92,57 |
| 0,4 (1,09 mmol) | 0,5 | F | 30ml acetone | r.t. | 23,5 | 4,0 | 95,9 |
| 0,4 (1,09 mmol) | 0,5 | C | 50 ml acetonitrile | r.t. | 31,5 | 3,07 | 96,92 |
| 0,4 (1,09 mmol) | 1 | C | 50 ml acetonitrile | r.t. | 78,25 | 39,01 | 60,98 |
| 0,4 (1,09 mmol) | 0,5 | C | 15 ml ethylacetate | r.t. | 14,9 | 3,62 | 96,37 |
| 0,4 (1,09 mmol) | 1 | C | 27 ml ethylacetate | r.t. | 35 | 7,78 | 92,21 |
| 0,4 (1,09 mmol) | 1 | F | 4ml dioxane | r.t. | 21 | 33,5 | 66,4 |

### Resolution with R-(+)-1-Phenylethylamine

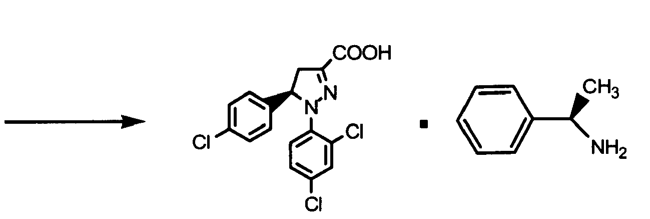

| Acid | Eq. amine | Proc. | Solvent for crystallisation | T Cryst. | Yield 1^{st} Cryst. % | % S-Acid | % R-Acid |
|---|---|---|---|---|---|---|---|
| 0,4 (1,09 mmol) | 1 | D | 1,6ml etanol | r.t. | 22 | 51,4 | 48,6 |
| 0,4 (1,09 mmol) | 0,5 | E | 6ml acetonitrilo | r.t. | 11 | 8,1 | 91,9 |
| 0,4 (1,09 mmol) | 1 | E | 6ml acetonitrilo | r.t. | 50 | 36,8 | 63,2 |
| 0,4 (1,09 mmol) | 1 | E | 6ml dioxano | ≈5°C | 5 | 4,6 | 95,3 |

### Detailed description:

### Resolution of 5-(4-chlorophenyl)-1-(2,4-dichlorophenyl)-4,5-dihydro-pyrazole-3-carboxylic acid with (+)-Cinchonine

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystalize at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallisation of the diastereomeric salt was carried out in different solvents in order to improve the enantiomeric excess:

| Solvent | Yield | ratio of enantiomers % (R) / % (S) |
|---|---|---|
| Acetonitrile | 90 % | 99,6 / 0,4 |
| EtOH-H₂O | 68,5 % | 99,3 / 0,7 |
| Isopropanol | 55 % | 98,9/1,1 |

Consequently, the product was recrystallized from acetonitrile to give 22.6 g of crystals (yield 72 mol-% related to chiral base). The ratio of enantiomers determined by capilar electrophoresis was:
99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine ee = 99.4 %.

### Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.

Enantiomeric excess determined by capilar electrophoresis. ee = 99.2 %

Chemical purity determined by HPLC: 99,3 %

[□]_{D} (c=1,23°C, MeOH) = -429.

### Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine was treated with 6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).

Enantiomeric excess as determined by capilar electrophoresis was ee = 92 %.

The purity of the isolated salt was comparable to the one of the salt obtained directly from the resolution of the racemate. Consequently, the same purification process as described above could be applied.

### Resolution of racemic 5-(4-chlorophenyl)-1-(2,4-dichlorophenyl)-4,5-dihydro-pyrazole-3-carboxylic acid with Brucine

Preparation of the diastereomeric salt with Brucine

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallize for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:
99 % of (S)-5-(4-chlorophenyl)-1-(2,4-dichlorophenyl)-4,5-dihydro-pyrazole-3-carboxylic acid Brucine
1 % of (R)-5-(4-chlorophenyl)-1-(2,4-dichlorophenyl)-4,5-dihydro-pyrazole-3-carboxylic acid Brucine
Enantiomeric excess ee = 98 %

The filtered mother liquors were left in the refrigerator and yielded a second fraction of crystals, which were filtered off and dried to give another 1.56 g (5.1 molar % related to the base).

The analysis of the 2^{nd} fraction via capillar electrophoresis gave 99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %

Total yield:
15.22 g (50.1 mol% related to base) of the sal with ee = 98 %.

### Determination of the chirality of the Brucine salt via X-ray crystallography

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

### Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with column chromatography (silical gel, Cl₂CH₂:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 °C.

CCF(Cl₂CH₂:MeOH 95:5) : R_{f} = 0.3
The analysis of the enantiomers via capillar electrophoresis gave 99.1 % (S)-5-(4-chlorophenyl)-1-(2,4-dichlorophenyl)-4,5-dihydro-pyrazole-3-carboxylic acid
0.9 % (R)-5-(4-chlorophenyl)-1-(2,4-dichlorophenyl)-4,5-dihydro-pyrazole-3-carboxylic acid

Enantiomeric excess ee = 98.2 %

[□]_{D} (c=1,23°C, MeOH) = -480.5.

Chemical purity determined by HPLC: 99.1 %

¹H-NMR (CDCl₃) δ ppm: 3,2 (dd, J=6,4 y 18,2 Hz, 1 H), 3,7 (dd, J=12,7 y 18,2 Hz, 1 H), 5,85 (dd, J=6,4 y 12,6 Hz, 1 H), 7,0-7,2 (m, 7H).

### Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chlorophenyl)-1-(2,4-dichlorophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for a couple of minutes and left to crystallize at room temperature overnight. A creme-coloured solid was obtained that was filtered off and dried to yield 28 g (77.5 % relative to the (S)-salt).

### Enantiomeric excess as determined by capilar electrophoresis was ee = 98.2 %

The purity of the isolated salt was comparable to the one of the salt obtained directly from the resolution of the racemate. Consequently, the same purification process as described above could be applied.

The Examples 1 to 6 represent compounds according to formula X.

### Example 1:

### N-piperidinyl-5-(4-chlorophenyl)-1-(2,4-dichlorophenyl)-4,5-dihydro-1H-pyrazole-3-carboxamide

### (a) 5-(4-chlorophenyl)-1-(2,4-dichlorophenyl)-4,5-dihydro-pyrazole-3-carboxylic acid chloride

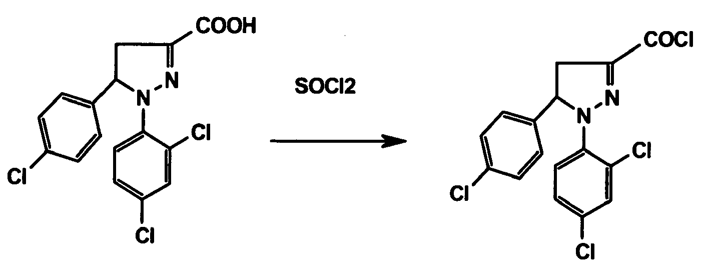

Under nitrogen atmosphere 5-(4-chlorophenyl)-1-(2,4-dichlorophenyl)-4,5-dihydro-pyrazole-3-carboxylic acid (2.5 g, 6.8 mmols) obtained according to Example 0 was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, cm⁻¹) : 1732,3, 1700, 1533,3, 1478,1, 1212,9, 826,6.

Starting from this compound compounds according to general formulas I and II wherein R¹ is a linear or branched C₁₋₄-alkyl group can be prepared reacting this compound with the appropriate alkyl alcohol.

### (b) N-piperidinyl-5-(4-chlorophenyl)-1-(2,4-dichlorophenyl)-4,5-dihydropyrazole-3-carboxamide

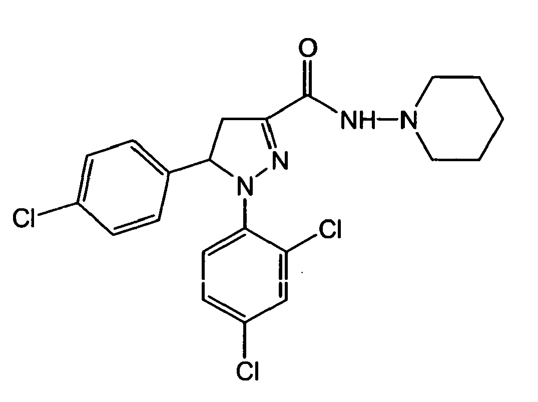

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (a) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, cm⁻¹) : 3222,9, 2934,9, 1647,4, 1474,7, 1268,3, 815,6.
¹H NMR (CDCl₃, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1H), 5,7 (dd, J=6,1 and 12,5 Hz, 1H), 7,0-7,2 (m, 6H), 7,4 (s, 1H).

The compounds according to the following examples 2-6 have been prepared analogously to the process described in Example 1 in combination with Example 0.

### Example 2:

### 5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 °C.
IR (KBr, cm⁻¹): 3448, 1686, 1477, 1243, 1091, 821.
¹H NMR(CDCl₃, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1 H).

### Example 3:

### 5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155°C.
IR (KBr, cm⁻¹) : 3433, 1685, 1477, 1296, 1246, 1088, 1014, 825.
¹H NMR (CDCl₃, δ): 2,7 (d, J=4,2Hz, 3H), 3,0-3,4 (m, 9H), 3,6 (dd, J=11,9 and
17,9 Hz, 1 H), 5,8 (dd, J=5,5 and 11,9 Hz, 1 H), 7,1 (d, J=8,4Hz, 2H), 7.25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

### Example 4:

### 5-(4-Chloro-phenyl)-1-(2,4-dichloro-phenyl)-4,5-dihydro-1H-pyrazole-3-carboxylic acid diethylamide

This compound was obtained in form of an oil.

IR (film, cm⁻¹): 2974, 1621, 1471, 1274, 1092, 820.
¹H NMR (CDCl₃, δ): 1,2 (m, 6H), 3,3-3,9 (m, 6H), 5,6 (dd, J=5,8 and 11,7 Hz, 1 H), 7-7,25 (m, 7H).

### Example 5:

### [5-(4-Chloro-phenyl)-1-(2,4-dichloro-phenyl)-4,5-dihydro-1H-pyrazol-3-yl]-piperidin-1-yl-methanone

Melting point: 105-110°C.
IR (KBr, cm⁻¹) : 2934, 1622, 1470, 1446, 1266, 1010, 817.
¹H NMR (CDCl₃, δ): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1H), 7-7,25 (m, 7H).

### Example 6:

### N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, cm⁻¹): 1697, 1481, 1436, 1340, 1169, 1074, 853.
¹H NMR (CDCl₃, δ): 2,4 (s, 3H), 3,2 (dd, J=6,6 and 18,3Hz, 1 H), 3,6 (dd, J=12,8 and
18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1 H).

### Example 7:

### N-oxide of (R)-N-piperidinyl-5-(4-chlorophenyl)-1-(2,4-dichlorophenyl)-4,5-dihydropyrazole-3-carboxamide

Under nitrogen gas as an inert atmosphere (R)-N-piperidinyl-5-(4-chlorophenyl)-1-(2,4-dichlorophenyl)-4,5-dihydropyrazole-3-carboxamide (0,15 g, 332 mmoles) (according to example 0) is dissolved in 7 ml of dichloromethane. The resulting solution is ice-cooled to 0 °C and m-chloroperbenzoic acid (0,204 g, 0,83 mmoles) added in several portions. After stirring for 15 minutes a control via thin layer chromatography shows that no starting material is remaining. A saturated solution of sodium bicarbonate is then slowly added, the organic phase separated, washed with water, dried over sodium sulfate and filtered. The filtered solution is evaporated to dryness and the crude product is purified via column chromatography yielding 78 mg (50 % of theoretical yield) of the N-oxide of (R)-N-piperidinyl-5-(4-chlorophenyl)-1-(2,4-dichlorophenyl)-4,5-dihydropyrazole-3-carboxamide in form of a white solid.

### Pharmacological Data/Testing:

The compound according to example 0 is an inhibitor of high blood levels of triglicerides. This effect is probed in obese mice fed with high fat diet. In the following paragraphs the method of this study is described.

### I. In-vivo testing for regulation of triglycerides in blood plasma

The study is done using six weeks old male mice B6 Lep ob/ob, obtained from Charles River (France). Mice are divided in 3 groups: I (control), II (vehicle), III (example 0).

### Group I:

The animals of the group I receive the standard diet (D-12450B, Research Diets, NJ, USA).

### Group II:

The animals of the groups II and III are fed with a High Fat Diet (D-12492, Research Diets, NJ, USA), in both cases for 7 weeks (References 1 and 2).

### Group III:

The animals of the groups III are fed with a High Fat Diet (D-12492, Research Diets, NJ, USA), in both cases for 7 weeks (References 1 and 2).

At the end of the feeding period of 7 weeks, the treatment period (14 days) is started: Group II mice receive the vehicle (10 ml/kg/day, po, of the aqueous solution of acacia gum, 5% W/V). Group III is administered with 30 mg/kg/day, p.o., of the inventive compound (R)-5-(4-chlorophenyl)-1-(2,4-dichlorophenyl)-4,5-dihydro-pyrazole-3-carboxylic acid according to Example 0. Group I does not receive any treatment. The three groups of mice have the same diet than in the previous period.

At the end of the 14 days period of treatment, the blood levels of triglicerides of the animals are determined.

The analysis of the whole blood samples is done using test strips "Lipid panel" and the photometric Analyzer Cardio-Check Test System, from PA Instruments Polymer

Technology Systems Indianapolis, IN-46268, USA (Distributed in Spain by Novalab Iberica S.A.L, Madrid, Spain).

### References

1.-Lambert P.D. et al.."Cyliary neurotrophic factor activates leptin-like pathways and reduces body fat" P.N.A.S. 2001, 28 (8) : 4652-4657
2.- Grasa M.M. et al "Oleoyl-Estrone lowens the body weight of both ob/ob and db/db mice. Hozm. Metab. Res 2000, 32 : 246-250

### II. In-vivo testing for regulation of triglycerides in blood plasma

In a second set of experiments similar to the tests shown above the TG (triglyceride) levels of diet-induced obese mice in blood is determined.
Mice receiving a high fat diet are - after a feeding period of 6 days - either treated p.o. with vehicle (0,5 % HPMC) or with the compound according to example 0 (30 mg/kg/day p.o.).

TG levels in blood are determined on day 28 after beginning of the treatment.

## Claims

1. Substituted pyrazoline compounds of general formula I, 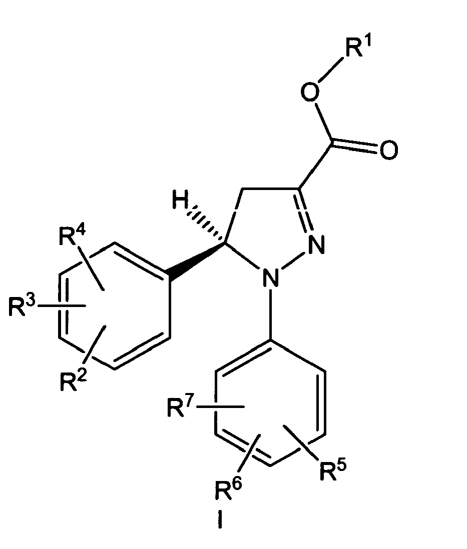 wherein
R¹ represents hydrogen or a linear or branched, substituted or unsubstituted, saturated or unsaturated C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵ and R⁶ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl; R⁷ represents hydrogen, a linear or branched C₁₋₆-alkyl group, a linear or branched C₁₋₆-alkoxy group, a halogen atom, CH₂F, CHF₂, CF₃, CN, OH, NO₂,-(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
with the proviso that
if R¹ and R⁷ are H and R⁵ and R⁶ both represent Cl in the 3- and 4-position of the phenyl ring neither of R², R³ and R⁴ may represent F in the 4-position of the phenyl ring if the other two of R², R³ and R⁴ both represent H;
optionally in form of a corresponding N-oxide thereof, or a corresponding salt thereof, or a corresponding solvate thereof.

2. Compounds according to claim 1, **characterized in that** at least one of R², R³ or R⁴ represents hydrogen, while at least one of R², R³ or R⁴ is different from hydrogen.

3. Compounds according to any one of claims 1 or 2, **characterized in that** R⁷ represents hydrogen.

4. Compounds according to any one of claims 1 to 3, **characterized in that** R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

5. Compounds according to any one of claims 1 to 4, **characterized in that** R⁵ and R⁶ independently of each other represent a linear or branched C₁₋₆-alkyl group, a halogen atom, or CF₃, preferably R⁵ and R⁶ independently of each other represent methyl, ethyl, F, Cl, Br and CF₃.

6. Compounds according to any one of claims 1 to 5, **characterized in that** R² represents a chlorine atom in the 4-position of the phenyl ring, while R³ and R⁴ represent hydrogen.

7. Compounds according to any one of claims 1 to 6, **characterized in that** R⁵ and R⁶ each represent a chlorine atoms in the 2- and 4-position of the phenyl ring, while R⁷ represents hydrogen.

8. Compounds according to any one of claims 1 to 7, **characterized in that** R¹ represents hydrogen, methyl or ethyl, preferably hydrogen.

9. Compounds of general formula II according to one or more of claims 1 to 8 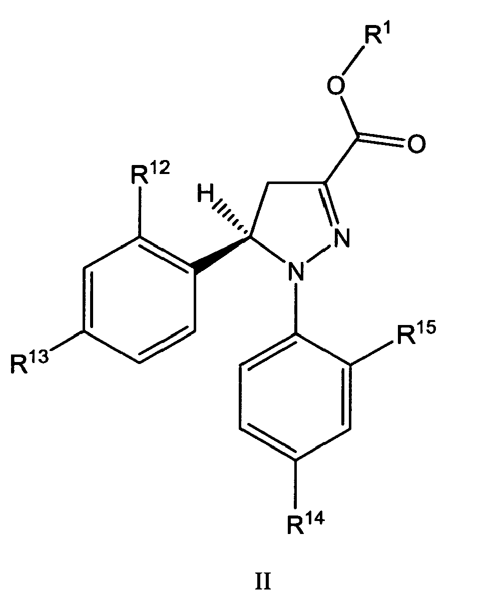 wherein
R¹ represents hydrogen or a linear or branched, substituted or unsubstituted, saturated or unsaturated, C₁₋₄-alkyl group,
R¹² or R¹³ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, methyl, ethyl, F, Cl, Br and CF₃,
optionally in form of a corresponding N-oxide thereof, or a corresponding salt thereof, or a corresponding solvate thereof.

10. Compounds according to claim 9 **characterized in that** R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

11. Compounds according to any one of claims 9 or 10, **characterized in that** R¹⁴, and R¹⁵ independently of each other represent a linear or branched C₁₋₆-alkyl group, a halogen atom, or CF₃, preferably R¹⁴ and R¹⁵ independently of each other represent methyl, ethyl, F, Cl, Br and CF₃.

12. Compounds according to any one of claims 9 to 11, **characterized in that** R¹³ represents Cl and R¹² represents hydrogen.

13. Compounds according to any one of claims 9 to 12, **characterized in that** R¹⁴ and R¹⁵ each represent Cl.

14. Compounds according to any one of claims 9 to 13, **characterized in that** R¹ represents hydrogen, methyl or ethyl, preferably hydrogen.

15. Compounds according to one or more of claims 1 to 14 selected from the group consisting of:
(R)-5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.

16. Combination of compounds comprising at least one a substituted pyrazoline compound of general formula I 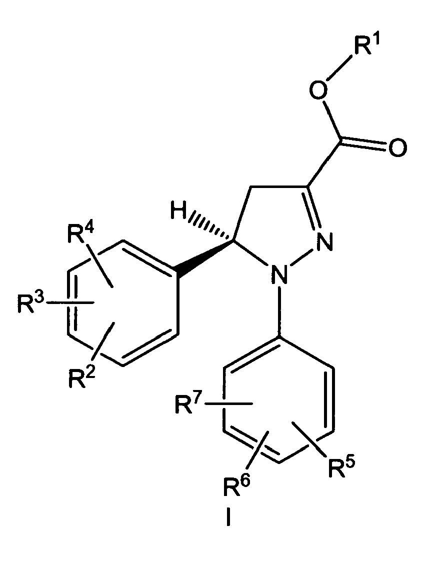 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl, R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof:
and at least one substituted pyrazoline compound of general formula X 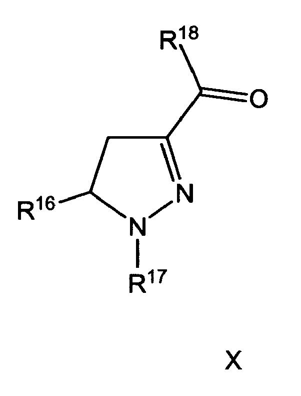 wherein
R¹⁶ represents an optionally at least mono-substituted phenyl group,
R¹⁷ represents an optionally at least mono-substituted phenyl group,
R¹⁸ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system, or an ―NR¹⁹R²⁰-moiety,
R¹⁹ and R²⁰, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R²¹-moiety, or an ―NR²²R²³-moiety, with the proviso that R¹⁹ and R²⁰ do not identically represent hydrogen,
R²¹ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R²² and R²³, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

17. Combination of compounds according to claim 16, **characterized in that** at least one of R², R³ or R⁴ represents hydrogen, while at least one of R², R³ or R⁴ is different from hydrogen.

18. Combination of compounds according to any one of claims 16 or 17, **characterized in that** at least on of R⁵, R⁶ or R⁷ represents hydrogen, while at least one R⁵, R⁶ or R⁷ is different from hydrogen.

19. Combination of compounds according to any one of claims 16 to 18, **characterized in that** R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

20. Combination of compounds according to any one of claims 16 to 19, **characterized in that** R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃.

21. Combination of compounds according to any one of claims 16 to 20, **characterized in that** R² represents a chlorine atom in the 4-position of the phenyl ring, while R³ and R⁴ represent hydrogen.

22. Combination of compounds according to any one of claims 16 to 21, **characterized in that** R⁵ and R⁶ each represent a chlorine atoms in the 2- and 4-position of the phenyl ring, while R⁷ represents hydrogen.

23. Combination of compounds according to any one of claims 16 to 22, **characterized in that** R¹ represents hydrogen, methyl or ethyl, preferably hydrogen.

24. Combination of compounds according to any one of claims 16 to 23 **characterized in that** the a compound of general formula I is represented by a compound of general formula II 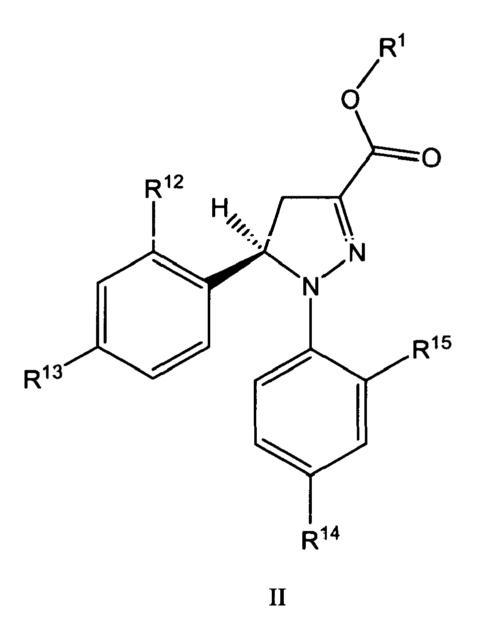 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof.

25. Combination of compounds according to claim 24 **characterized in that** R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

26. Combination of compounds according to any one of claims 24 or 25, **characterized in that** R¹⁴, and R¹⁵ independently of each other represent hydrogen, a linear or branched C₁₋₆-alkyl group, a halogen atom, or CF₃, preferably R¹⁴ and R¹⁵ independently of each other represent hydrogen, methyl, ethyl, F, Cl, Br and CF₃.

27. Combination of compounds according to any one of claims 24 to 26, **characterized in that** R¹³ represents Cl and R¹² represents hydrogen.

28. Combination of compounds according to any one of claims 24 to 27, **characterized in that** R¹⁴ and R¹⁵ each represent Cl.

29. Combination of compounds according to any one of claims 24 to 28, **characterized in that** R¹ represents hydrogen, methyl or ethyl, preferably hydrogen.

30. Combination of compounds according to one or more of claims 16 to 29 **characterized in that** the compound according to formula I or II selected from the group consisting of:
(R)-5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.

31. Combination of compounds according to any of claims 16 to 30, **characterized in that** R¹⁶ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R¹⁶ represents a phenyl group, which is optionally substituted by one or more substituents selected from the group consisting of methyl, ethyl, F, Cl, Br and CF₃, more preferably R¹⁶ represents a phenyl group, which is mono-substituted with a chlorine atom in the 4-position.

32. Combination of compounds according to any of claims 16 to 31, **characterized in that** R¹⁷ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R¹⁷ represents a phenyl group, which is optionally substituted by one or more substituents independently selected from the group consisting of methyl, ethyl, F, Cl, Br and CF₃, more preferably R¹⁷ represents a phenyl group, which is di-substituted with two chlorine atoms in its 2- and 4-position.

33. Combination of compounds according to one or more of claims 16 to 32, **characterized in that** R¹⁸ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, or an -NR¹⁹R²⁰-moiety, preferably R¹⁸ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or an ―NR¹⁹R²⁰-moiety, more preferably R¹⁸ represents a pyrrolidinyl group, a piperidinyl group or a piperazinyl group, whereby each of these groups may be substituted with one or more C₁₋₆-alkyl groups, or an ―NR¹⁸R¹⁹-moiety.

34. Combination of compounds according to one or more of claims 16-33, **characterized in that** R¹⁹ and R²⁰, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an ―SO₂-R²¹-moiety, or an ―NR²²R²³-moiety, preferably one of these residues R¹⁹ and R²⁰ represents a hydrogen atom and the other one of these residues R¹⁹ and R²⁰ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an-SO₂-R²¹-moiety, or an -NR²²R²³-moiety, or R¹⁹ and R²⁰, identical or different, each represent a C₁₋₆ alkyl group, more preferably one of these residues R¹⁹ and R²⁰ represents a hydrogen atom and the other one of these residues R¹⁹ and R²⁰ represents an optionally at least mono-substituted pyrrolidinyl group, an optionally at least mono-substituted piperidinyl group, an optionally at least mono-substituted piperazinyl group, an optionally at least mono-substituted triazolyl group, an ―SO₂-R²¹-moiety, or an ―NR²²R²³-moiety, or R¹⁹ and R²⁰, identical or different, represent a methyl group, an ethyl group, an n-propyl group, an isopropyl group, an n-butyl group, a sec-butyl group or a tert.-butyl group.

35. Combination of compounds according to one or more of claims 16-34, **characterized in that** R²¹ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R²¹ represents a C₁₋₆-alkyl group, a saturated, optionally at least mono-substituted cycloaliphatic group, which may be condensed with a mono- or polycyclic ring-system, or a phenyl group, which is optionally substituted with one or more C₁₋₆ alkyl groups.

36. Combination of compounds according to one or more of claims 16-35, **characterized in that** R²² and R²³, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R²² and R²³, identical or different, represent a hydrogen atom or a C₁₋₆ alkyl radical.

37. Combination of compounds according to any of claims 16 to 36 **characterized in that** the compound according to general formula X is represented by a structure wherein
R¹⁶ represents a phenyl ring, which is mono-substituted with a halogen atom, preferably a chlorine atom, in its 4-position,
R¹⁷ represents a phenyl ring, which is di-substituted with two halogen atoms, preferably chlorine atoms, in its 2- and 4-position,
R¹⁸ represents a pyrrolidinyl group, a piperidinyl group, a piperazinyl group, a homo-piperazinyl group, a morpholinyl group, or an ―NR¹⁹R²⁰-moiety,
R¹⁹ represents a hydrogen atom or a linear or branched C₁₋₆-alkyl group,
R²⁰ represents a linear or branched C₁₋₆ alkyl group, an -SO₂-R²¹-moiety, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, and
R²¹ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

38. Combination of compounds according to one or more of claims 16 to 37, **characterized in that** it comprises at least one compound according to formula X selected from the group consisting of:
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

39. Combination of compounds according to one or more of claims 16 to 38, **characterized in that** it comprises at least
• (R)-5-(4-chloro-phenyl)-1-(2,4-dichlorophenyl)-4,5-dihydro-1H-pyrazol-3-carboxylic acid,
and
• (R)-N-piperidinyl-5-(4-chloro-phenyl)-1-(2,4-dichlorophenyl)-4,5-dihydro-1 H-pyrazol-3-carboxamide; or
• (S)-N-piperidinyl-5-(4-chloro-phenyl)-1-(2,4-dichlorophenyl)-4,5-dihydro-1 H-pyrazol-3-carboxamide; or
• mixtures of these, especially
• (rac)-N-piperidinyl-5-(4-chloro-phenyl)-1-(2,4-dichlorophenyl)-4,5-dihydro-1 H-pyrazol-3-carboxamide;
each optionally in the form of a corresponding N-oxide, a corresponding salt or a corresponding solvate.

40. Process for the manufacture of substituted pyrazoline compounds of general formula I or II, wherein R1 is hydrogen, according to one or more of claims 1 to 15, **characterized in that** at least one benzaldehyde compound of general formula III 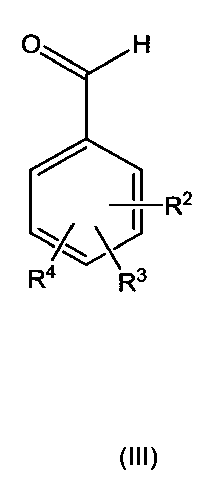 wherein R², R³ and R⁴ have the meaning according to one or more of claims 1-8, is reacted with a pyruvate compound of general formula (IV) 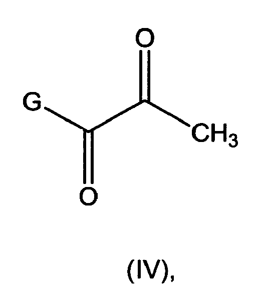 wherein G represents an OR group with R being a branched or unbranched C₁₋₆ alkyl radical or G represents an O⁻K group with K being a cation,to yield a compound of general formula (V) 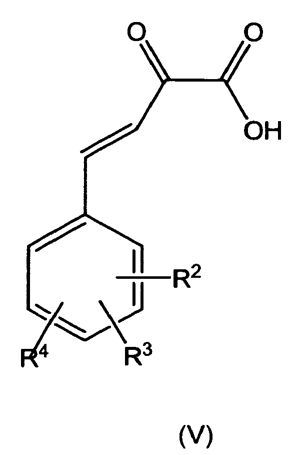 which is optionally isolated and/or optionally purified, and which is reacted with an optionally substituted phenyl hydrazine of general formula (VI) 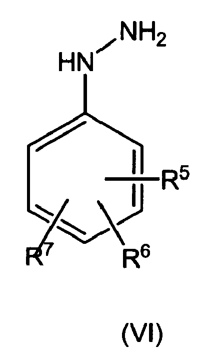 or a corresponding salt thereof, wherein R⁵, R⁶ and R⁷ have the meaning according to one or more of claims 1-8, under inert atmosphere, to yield a compound of general formula (VII) 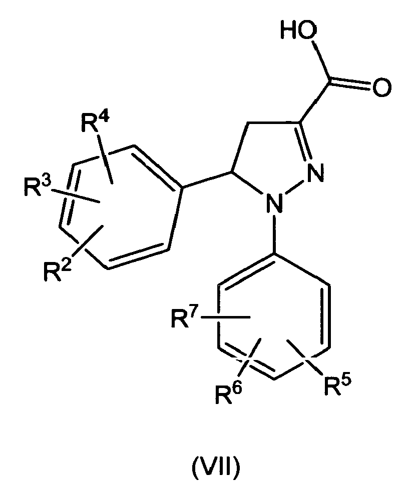 wherein R², R³, R⁴, R⁵, R⁶ and R⁷ have the meaning as given above, which is optionally isolated and/or optionally purified, and optionally esterified it to an alkyl-ester if in the substituted pyrazoline compound of general formula I according to one or more of claims 1 to 15 R¹ is a linear or branched, substituted or unsubstituted, saturated or unsaturated, C₁₋₄-alkyl group, achieving a compound of formula VII a 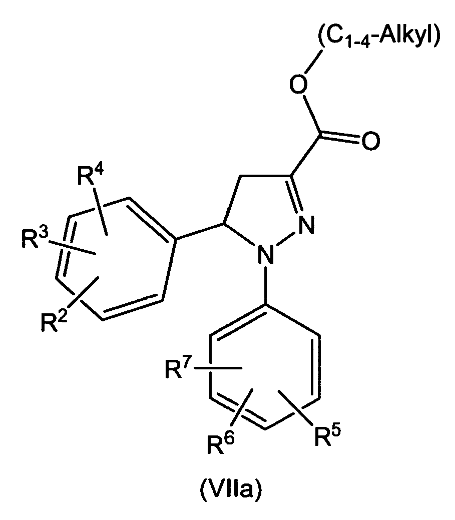 followed by reacting the resulting compound of general formula VII or VIIa with a suitable chiral base to produce a compound of general formula I or II.

41. Process according to claim 40, **characterized in that** the suitable chiral base is selected from (+)-Cinchonine or R-(+)-1-Phenylethylamine.

42. Medicament comprising at least one substituted pyrazoline compound of general formula I or II according to one or more of claims 1 to 15, and optionally one or more pharmaceutically acceptable excipients.

43. Medicament comprising at least one substituted pyrazoline compound of general formula I 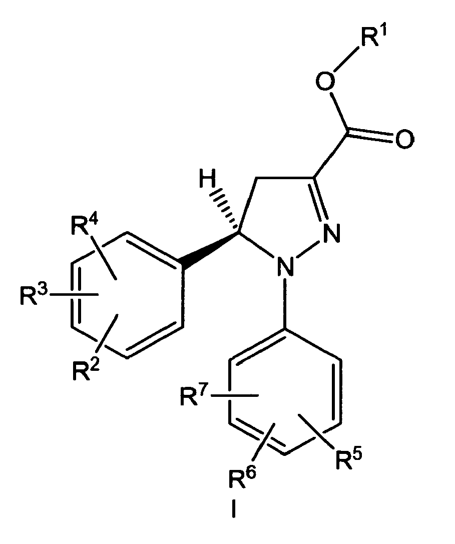 wherein
R¹ represents hydrogen or a linear or branched, substituted or unsubstituted, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸. NR⁸R⁹. -(C=O)-NH₂. -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof;
and optionally one or more pharmaceutically acceptable excipients.

44. Medicament according to claim 43, **characterized in that** at least one of R², R³ or R⁴ represents hydrogen, while at least one of R², R³ or R⁴ is different from hydrogen.

45. Medicament according to any one of claims 43 or 44, **characterized in that** at least on of R⁵, R⁶ or R⁷ represents hydrogen, while at least one R⁵, R⁶ or R⁷ is different from hydrogen.

46. Medicament according to any one of claims 43 to 45, **characterized in that** R², R³ and R⁴ independently of each other represent hydrogen, a linear or branched C_{1- 6}-alkyl group, a halogen atom, or CF₃, preferably R², R³ and R⁴ independently of each other represent hydrogen, methyl, ethyl, F, Cl, Br and CF₃.

47. Medicament according to any one of claims 43 to 46, **characterized in that** R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃.

48. Medicament according to any one of claims 43 to 47, **characterized in that** R² represents a chlorine atom in the 4-position of the phenyl ring, while R³ and R⁴ represent hydrogen.

49. Medicament according to any one of claims 43 to 48, **characterized in that** R⁵ and R⁶ each represent a chlorine atoms in the 2- and 4-position of the phenyl ring, while R⁷ represents hydrogen.

50. Medicament according to any one of claims 43 to 49, **characterized in that** R¹ represents hydrogen, methyl or ethyl, preferably hydrogen.

51. Medicament according to one or more of claims 43 to 50 **characterized in that** the compound of general formula (I) is represented by a compound of general formula (II) 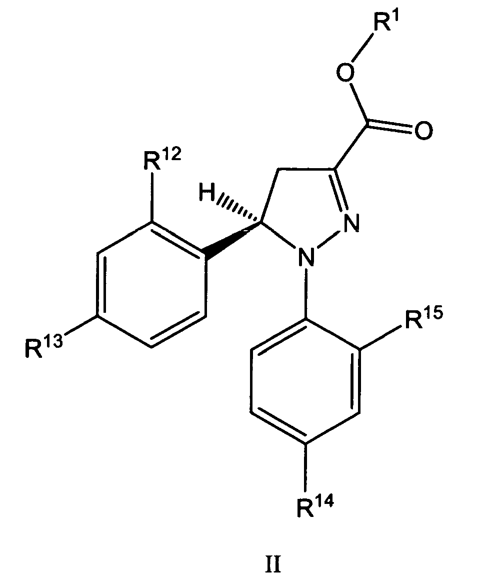 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof.

52. Medicament according to claim 51 **characterized in that** R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

53. Medicament according to any one of claims 51 or 52, **characterized in that** R¹⁴, and R¹⁵ independently of each other represent hydrogen, a linear or branched C₁-6-alkyl group, a halogen atom, or CF₃, preferably R¹⁴ and R¹⁵ independently of each other represent hydrogen, methyl, ethyl, F, Cl, Br and CF₃.

54. Medicament according to any one of claims 51 to 53, **characterized in that** R¹³ represents Cl and R¹² represents hydrogen.

55. Medicament according to any one of claims 51 to 54, **characterized in that** R¹⁴ and R¹⁵ each represent Cl.

56. Medicament according to any one of claims 51 to 55, **characterized in that** R¹ represents hydrogen, methyl or ethyl, preferably hydrogen.

57. Medicament according to one or more of claims 43 to 56 **characterized in that** the compound according to formulas I or II is selected from the group consisting of:
(R)-5-(4-chloro-phenyl)-1-(2,4-dichlorophenyl)-4,5-dihydro-1 H-pyrazol-3-carboxylic acid,
optionally in the form of a corresponding N-oxide, a corresponding salt or a corresponding solvate.

58. Medicament comprising at least one combination of compounds according to one or more of claims 16 to 39 and optionally one or more pharmaceutically acceptable excipients.

59. Medicament according to claim 43 to 58 for the regulation of triglyceride levels in the blood plasma and for the prophylaxis and/or treatment of disorders of the central nervous system, especially stroke, of disorders of the cardiovascular system and of of food intake disorders, preferably bulimia, anorexia, cachexia, obesity, type II diabetus mellitus (non-insuline dependent diabetes mellitus), preferably obesity and diabetis.

60. Medicament according to one or more of claims 43 to 57 for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

61. Medicament according to claim 58 for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

62. Medicament according to one or more of claims 43 to 58 for the prophylaxis and/or treatment of food intake disorders, preferably bulimia, anorexia, cachexia, obesity, type II diabetus mellitus (non-insuline dependent diabetes mellitus), preferably obesity.

63. Medicament according to one or more of claims 43 to 58 for the prophylaxis and/or treatment of psychosis.

64. Medicament according to one or more of claims 43 to 58 for the prophylaxis and/or treatment of alcohol abuse and/or addiction, nicotine abuse and/or addiction, drug abuse and/or addiction and/or medicament abuse and/or addiction, preferably drug abuse and/or addiction and/or nicotine abuse and/or addiction.

65. Medicament according to one or more of claims 43 to 58 for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

66. Use of at least one substituted pyrazoline compound according to one or more of claims 1 - 15 or at least one combination of compounds according to one or more of claims 16 to 39 and optionally one or more pharmaceutically acceptable excipients, for the preparation of a medicament for the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

67. Use of at least one substituted pyrazoline compound according to one or more of claims 1 - 15 and optionally one or more pharmaceutically acceptable excipients, for the preparation of a medicament for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

68. Use of at least one combination of compounds according to one or more of claims 16 to 39 and optionally one or more pharmaceutically acceptable excipients, for the preparation of a medicament for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

69. Use of at least one substituted pyrazoline compound according to one or more of claims 1-15 or at least one combination of compounds according to one or more of claims 16 to 39 and optionally one or more pharmaceutically acceptable excipients, for the preparation of a medicament for the prophylaxis and/or treatment of food intake disorders, preferably bulimia, anorexia, cachexia, obesity, type II diabetus mellitus (non-insuline dependent diabetes mellitus), preferably obesity.

70. Use of at least one substituted pyrazoline compound according to one or more of claims 1-15 or at least one combination of compounds according to one or more of claims 16 to 39 and optionally one or more pharmaceutically acceptable excipients, for the preparation of a medicament for the prophylaxis and/or treatment of psychosis.

71. Use of at least one substituted pyrazoline compound according to one or more of claims 1-15 or at least one combination of compounds according to one or more of claims 16 to 39 and optionally one or more pharmaceutically acceptable excipients, for the preparation of a medicament for the prophylaxis and/or treatment of alcohol abuse and/or addiction, nicotine abuse and/or addiction, medicament abuse and/or addiction and/or drug abuse and/or addiction, preferably drug abuse and/or addiction or nicotine abuse and/or addiction.

72. Use of at least one substituted pyrazoline compound according to one or more of claims 1-15 or at least one combination of compounds according to one or more of claims 16 to 39 and optionally one or more pharmaceutically acceptable excipients, for the preparation of a medicament for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

73. Use of at least one substituted pyrazoline compound of general formula I 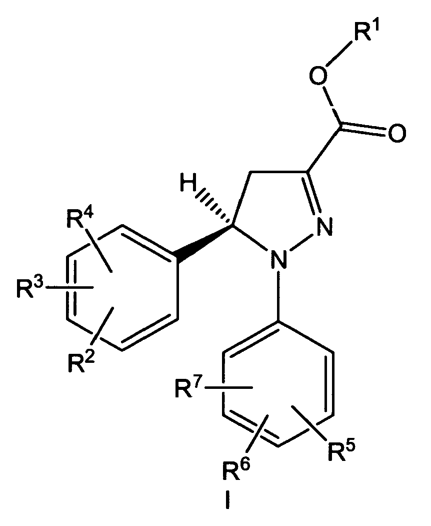 wherein
R¹ represents hydrogen or a linear or branched, substituted or unsubstituted, saturated or unsaturated, C₁₋₄-alkyl group,
R², R³ and R⁴ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R⁸, SH, SR⁸, SOR⁸, SO₂R⁸, NH₂, NHR⁸, NR⁸R⁹, -(C=O)-NH₂, -(C=O)-NHR⁸ or -(C=O)-NR⁸R⁹ whereby R⁸ and R⁹ for each substituent independently represent linear or branched C₁₋₆ alkyl,
R⁵, R⁶ and R⁷ independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁰, SH, SR¹⁰, SOR¹⁰, NH₂, NHR¹⁰, NR¹⁰R¹¹, -(C=O)-NH₂, -(C=O)-NHR¹⁰ and -(C=O)-NR¹⁰R¹¹, whereby R¹⁰ and optionally R¹¹ for each substituent independently represent linear or branched C₁₋₆ alkyl;
optionally in form of a corresponding N-oxide thereof, or a corresponding salt thereof, or a corresponding solvate thereof;
and optionally one or more pharmaceutically acceptable excipients, for the preparation of a medicament for the regulation of triglyceride levels in the blood plasma and for the prophylaxis and/or treatment of disorders of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

74. Use according to claim 73, **characterized in that** at least one of R², R³ or R⁴ represents hydrogen, while at least one of R², R³ or R⁴ is different from hydrogen.

75. Use according to any one of claims 73 or 74, **characterized in that** at least on of R⁵, R⁶ or R⁷ represents hydrogen, while at least one R⁵, R⁶ or R⁷ is different from hydrogen.

76. Use according to any one of claims 73 to 75, **characterized in that** R², R³ and R⁴ independently of each other represent hydrogen, a linear or branched C₁₋₆-alkyl group, a halogen atom, or CF₃, preferably R², R³ and R⁴ independently of each other represent hydrogen, methyl, ethyl, F, Cl, Br and CF₃.

77. Use according to any one of claims 73 to 76, **characterized in that** R⁵, R⁶ and R⁷ independently of each other represent hydrogen, a linear or branched C₁₋₆-alkyl group, a halogen atom, or CF₃, preferably R⁵, R⁶ and R⁷ independently of each other represent hydrogen, methyl, ethyl, F, Cl, Br and CF₃.

78. Use according to any one of claims 73 to 77, **characterized in that** R² represents a chlorine atom in the 4-position of the phenyl ring, while R³ and R⁴ represent hydrogen.

79. Use according to any one of claims 73 to 77, **characterized in that** R⁵ and R⁶ each represent a chlorine atoms in the 2- and 4-position of the phenyl ring, while R⁷ represents hydrogen.

80. Use according to any one of claims 73 to 79, **characterized in that** R¹ represents hydrogen, methyl or ethyl, preferably hydrogen.

81. Use according to one or more of claims 73 to 80 **characterized in that** the compound of general formula (I) is represented by a compound of general formula (II) 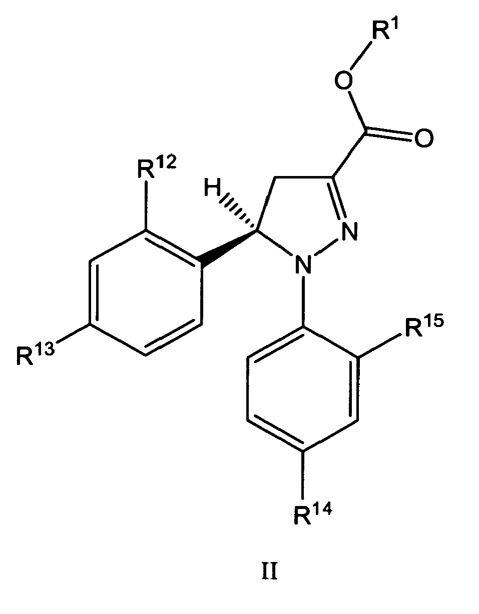 wherein
R¹ represents hydrogen or a linear or branched, substituted or unsubstituted, saturated or unsaturated, C₁₋₄-alkyl group,
R¹², R¹³, R¹⁴ or R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, SH, NH₂, hydrogen, methyl, ethyl, F, Cl, Br and CF₃,
optionally in form of a corresponding N-oxide thereof, or a corresponding salt thereof, or a corresponding solvate thereof.

82. Use according to claim 81 **characterized in that** R¹² and R¹³ independently of each other represent hydrogen, a linear or branched C₁₋₆-alkyl group, a halogen atom, or CF₃, preferably R¹² and R¹³ independently of each other represent hydrogen, methyl, ethyl, F, Cl, Br and CF₃.

83. Use according to any one of claims 81 or 82, **characterized in that** R¹⁴, and R¹⁵ independently of each other represent hydrogen, a linear or branched C₁₋₆-alkyl group, a halogen atom, or CF₃, preferably R¹⁴ and R¹⁵ independently of each other represent hydrogen, methyl, ethyl, F, Cl, Br and CF₃.

84. Use according to any one of claims 81 to 83, **characterized in that** R¹³ represents Cl and R¹² represents hydrogen.

85. Use according to any one of claims 81 to 84, **characterized in that** R¹⁴ and R¹⁵ each represent Cl.

86. Use according to any one of claims 81 to 85, **characterized in that** R¹ represents hydrogen, methyl or ethyl, preferably hydrogen.

87. Use according to one or more of claims 73 to 86 **characterized in that** the compound according to formulas I or II is selected from the group consisting of:
(R)-5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.
